# EUROPEAN PATENT SPECIFICATION

(11) **EP 1 583 477 B1**
(45) Date of publication and mention of the grant of the patent: **01.07.2009**
(21) Application number: 02808275.8
(22) Date of filing: 23.12.2002
(51) Int. Cl.: A61B 17/22

(54) **SURGICAL DEVICE FOR EXTRACTING A FOREIGN OBJECT AND METHOD FOR MANUFACTURING THEREOF**
CHIRURGISCHES GERÄT ZUR ENTNAHME EINES FREMDKÖRPERS UND VERFAHREN ZU DESSEN HERSTELLUNG
DISPOSITIF CHIRURGICAL PERMETTANT D'EXTRAIRE UN OBJET ETRANGER ET PROCEDE DE FABRICATION DE CE DISPOSITIF

(43) Date of publication of application: 12.10.2005
(73) Proprietor: Lithotech Medical Ltd, 12900 Katzrin (IL)
(72) Inventor: KHACHIN, Vladimir, 634021 Tomsk (RU); KHACHIN, Stepan, 634021 Tomsk (RU); DIAMANT, Valery, 12900 Katzrin (IL)
(74) Representative: Neunert, Peter Andreas
(86) International application number: PCT/IL2002/001028
(87) International publication number: WO 2004/056275

(56) References cited:
- EP-A- 0 820 729
- WO-A-96/23446
- WO-A-02/078632
- WO-A-03/002006
- US-B1- 6 348 056
- US-B1- 6 458 139
- US-B1- 6 458 145

## Description

### FIELD OF THE INVENTION

This invention relates to a fabrication of a medical instrument for removing an object from a body, and in particular, to a fabrication of a surgical device for extracting calculi appearing in the biliary or urinary system.

### BACKGROUND OF THE INVENTION

One type of a widely used surgical device is a tool for retrieving foreign material from various sites along the urinary tract of a patient's body. Examples of the foreign material include calculi of different sizes and characteristics.

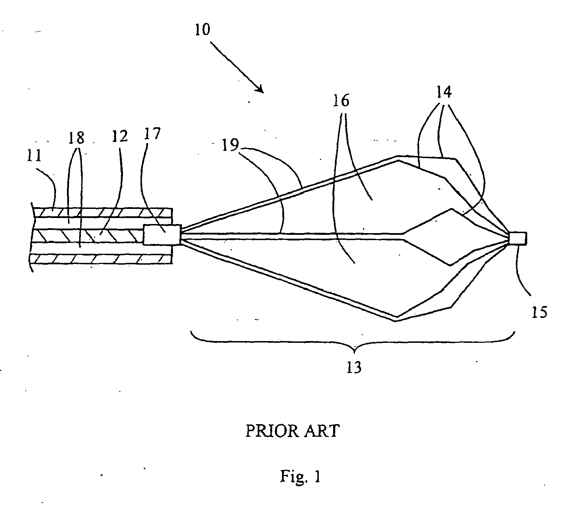

Referring to **Fig. 1**, a schematic view, partially in a longitudinal cross-section of the distal end of an example of a conventional surgical retrieval tool is illustrated. The surgical retrieval tool **10** or so-called surgical extractor, generally comprises a flexible tubular catheter **11** formed as a tubular sheath adapted to penetrate the body passages to reach the location where the object (not shown) to be evacuated is located. A manipulation rod or cable **12** is located within the catheter **11**, which can be manipulated from the outside at the catheter's proximal end. The rod **12** is coupled to a basket **13** capable to be deployed within the sheath. The basket **13** consists of flexible filaments **14** and, for example, can be made of either stainless steel or any other material capable to provide the basket with elasticity. According to the example shown in Fig. 1, the filaments **14** are bound together in the vicinity of a basket proximal end to form strands **19** as well as at a basket distal end to form a tip 15. The basket 13 is usually connected to the manipulation rod **12** via a sleeve connector **17** bounding the strands and filaments together.

One of the drawbacks of the conventional surgical extractor is associated with the discrepancy of the inner diameter of the catheter **11,** the diameter of the manipulating rod **12** and the diameter of the connector **17.** Due to this discrepancy, an air gap **18** between the inner wall of the catheter **11** and the rod **12** significantly affects deformation properties of the catheter and the ability of the catheter to bend without destruction on large angles.

Depending on the manipulation, the basket **13** may either retract inside the sheath to allow penetration of the catheter via a passage or protract from the catheter. In the protracted position, the filaments open due to the elasticity of their material and form a cage to thus allow entering the object inside the basket **13** through the open spaces **16** left between its adjacent filaments. Further retraction of the basket inside the sheath results in the cage collapsing and entrapping the object in the basket. Removal of the catheter will enable the whole device to be removed from the body organ together with the object immobilized within the basket.

During an operation, the surgeon moves the catheter behind the object to be extracted, and then protracts the basket from the catheter. Once the basket is protracted it opens (due to its resiliency) and is ready for receiving the object to be entrapped therein. The surgeon pulls the catheter together with the basket until it entraps the object, and thus extracts the entrapped object from the body.

It can be easily appreciated that the particular design of the basket is crucial for entrapping and reliably retaining the object during evacuation. Examples of various types of such retrieval baskets are described in the following documents: U.S. Pat. Nos. 2,943,626; 3,137,298; 3,472,230; 4,299,225; 4,347,846; 4,590,938; 4,633,871; 4,611,594; 4,790,812; 5,057,114; 5,496,330; 5,064,428; 5,658,296; 6,168,603; 6,183,482 and 6,190,394 as well as WO03/002006.

It should be noted that generally all conventional retrieval baskets have certain common characteristics. Thus, each retrieval basket comprises a plurality of filaments and can be collapsible into a compact form. The filaments are formed from wires and can be arranged into either multi-filament strands or spaced single filaments.

There are known the baskets that have relatively few widely spaced multi-filament strands at the proximal basket end, for capturing the object. These baskets may include two or more such multi-filament strands between which the objects can pass for entrapping within the retrieval basket. On the other hand, the filaments are closely spaced in the vicinity of the distal basket end, to provide an enclosure for retaining objects captured within such retrieval baskets. In some retrieval baskets the strands are formed along substantially straight lines when the basket is in compact form; in others, the individual strands extend along a generally helical path.

Forming the conventional baskets generally comprises grouping a plurality of axially extending filaments, strips or wires having shape memory or superelastic properties to extend along the basket axis and bounding them together at distal and proximal basket ends between which each of the wires extend.

In order to increase the number of contacts between the basket and entrapped calculi, additional filaments would be advantageous. However, an inner diameter of the catheter imposes an upper limit on the filament number and their diameter. Thus, for sufficient strength of the basket, the basket design involves a compromise between the number of filaments (needed to retain objects and enable the capture of the objects) and the wire diameter.

It can be appreciated that rigid wire materials can be employed in order to enhance the strength of the basket, However, the utilization of rigid materials can result in a decrease in flexibility that is necessary to provide penetration of the basket in body tracts having small diameters, tortuous pathways and irregular lumens.

International Application WO 96/23446, which provides the basis for the two-part form of claims 1 and 12, describes a surgical extractor for removing objects from a body, such as kidney stones and gall stones. The extractor includes a handle at a proximal end of the extractor with a slider for operation by a physician. At the distal end the extractor includes a plurality of wires with each wire comprising a first portion having an individual strand, and a second portion comprising a plurality of filaments. The use of such a basket promotes capture of objects within the body by having widely spaced wires in the first section, retention of such objects in the second section by multiplying the number of contacts with entrapped object, and improved selective release of such objects without any deleterious effect on the reliability or size of the extractor.

International Application WO 03/002006 is an Art. 54(3) EPC document for this invention. It describes a surgical device for removing a foreign object from a body. The device includes a retrieval basket for entrapping and retaining the object located in the body, a basket control assembly comprising a tubular sheath adapted to penetrate into the body for reaching the object, and a manipulator for manipulating the basket for extraction the object from the body. The basket comprises a structure having a proximal end and a distal end. The structure is formed by a plurality of filaments fabricated from a single or several wires. The filaments extend from the proximal end towards the distal end. At least a part of the filaments are configured in the form of loops. At least a part of the loops are interlaced so as to define a net at the distal end, and thereby impart structural rigidity and dilatation ability to the basket when opened.

### SUMMARY OF THE INVENTION

All passages of the description, including drawings, that are not within the scope of claims 1,12 or 25 do not form part of the claimed invention and are presented as examples useful for the understanding of the invention.

Despite the extensive prior art in the area of surgical devices employing retrieval baskets for extracting objects from a body and methods for manufacturing thereof, there is still a need in the art for, and it would be useful to have a convenient and safe surgical device suitable for the reliable and efficient extraction of foreign objects from the body as well as a novel and repeatable method for producing thereof.

It would be advantageous to have a repeatable method for producing a novel surgical device provided with a retrieval basket having enhanced structural rigidity and dilatation ability, thus reducing the probability of traumatizing of adjacent body tissues.

It would also be advantageous to have a repeatable method for producing a novel surgical tool provided with a catheter capable to withstand various bends on relatively large angles, thus enabling more reliable functioning during surgical treatment.

It would yet be advantageous to have a repeatable relatively inexpensive method for producing a retrieval basket from one piece of wire or from several wires.

The present invention satisfies the aforementioned need by providing a retrieval basket suitable for entrapping and retaining the object during extraction. The basket comprises a structure having a proximal end and a distal end. The structure is formed by a plurality of filaments fabricated from a single or several wires. The filaments extend between the proximal end and the distal end. At least a part of the filaments, which originate from the proximal end, can arrive at the proximal end after winding. At least a part of the filaments are configured in the form of loops. The filaments, from which the loops are made, can overlap and/or interlace so as to define a net, and thereby to impart structural rigidity and dilatation ability to the basket when it is opened.

Note that the term "overlap" herein is assigned to such arrangement of the filaments, in which one element crosses other filaments, i.e., one of the filaments always being over or under the other filaments. The term "interlace" herein is assigned to the situation when at least one filament interweaves with the other filaments, i.e., one of the filaments passes first above the crossed filament and then passes under the next crossed filament.

According to one embodiment of the basket of the invention, the size of holes (cells) of the net decreases from the proximal end towards the distal end. In other words, the density of the net increases from the proximal end to the distal end of the structure.

According to a further embodiment of the basket, the filaments in a region of the structure at the proximal end form at least two strands, each including a plurality of the filaments. According to this embodiment, the structure of the basket has a parachute-like shape, i.e., a form of the basket is symmetrical along the basket central axis.

According to a still further embodiment of the basket of the invention, the filaments in a region of the structure at the proximal end form a plurality of strands and one of the strands is common for all the filaments. The structure of the basket, according to this embodiment, has a spoon-like shape.

According to a yet further embodiment of the basket of the invention, the filaments are bound together at the distal ends to form a basket tip.

According to a still further embodiment of the basket of the invention, the filaments are bound together at the proximal end to form a manipulation cable.

According to another aspect, the invention relates to a production of a medical device for retrieving an object from a body. The medical device includes the retrieval basket of the present invention, a flexible tubular catheter (designed as a sheath) adapted to penetrate along the body passages near the location of the object, and a manipulator coupled to the retrieval basket via a manipulation cable. The catheter is suitable either for retracting the basket within the sheath, to enable bringing the catheter within the body, or for protracting the basket from the sheath to enable opening of the basket.

According to one embodiment of the medical device of the invention, the manipulation cable is produced as an integral part of the basket. In this case at least a part of the cable is formed from the basket's filaments which are bound together at the basket proximal end and expended towards the manipulator.

According to a further embodiment of the medical device, at least a portion of the manipulation cable is covered by a filling tubing. The outer diameter of the filling tubing has a sufficient magnitude to substantially fill the gap between the inner wall of the catheter and the manipulation cable, in order to enhance the ability of the catheter to bend without destruction on large angles.

According to a still another embodiment of the medical device of the invention, the manipulation cable is produced as a separate unit that is coupled to the manipulator and to the basket. The joint between the cable and the basket is accomplished by means of a bushing. For example, the bushing can be in the form of a pipe made of metal, e.g., Ni, stainless steel, etc.

The present invention also satisfies the aforementioned need by providing a process for manufacturing a retrieval basket suitable for entrapping the object and its retaining during the extraction. The process begins with a step of selection of a predetermined number of wires having a predetermined diameter and length.

According to one non-limiting example of the process, the manufacturing of the retrieval basket is carried out from one piece of wire.

According to another non-limiting example of the process the manufacturing of the retrieval basket is carried out from several wires.

It should be noted that the wires selected for the construction of the basket - can be single-filament wires, or when desired, can be multi-filament wires.

Furthermore, the process for fabrication of the retrieval basket includes weaving the retrieval basket. The weaving can be carried out from one piece of wire or from several wires.

According to one embodiment of the invention, at least a part of the weaving of the retrieval basket is performed on a weaving jig having a predetermined pattern formed by grooves configured on the jigs surface.

Thereafter, the process includes forming a shape of the retrieval basket. According to one embodiment of the invention, the forming of a shape includes binding the filaments on a shape-forming jig which for this .purpose has a predetermined shape. According to this embodiment, the forming of a shape further includes shape storing annealing the basket mounted on the shape-forming jig. Such forming a shape allows the basket to impart the structural rigidity and dilatation abilities, when the basket is opened. The weaving is such that one of the strands of the plurality of strands is common for all the wire filaments and forms a loop, and the retrieval basket is formed in a spoon-like shape.

The process for fabrication of the retrieval basket can include binding the filaments together at the proximal end to form a manipulation cable.

When required, the process can also include binding the filaments together at the distal end to form a basket tip.

Thus, in accordance with one broad aspect of the invention, there is provided a method for manufacturing a retrieval basket having a proximal end and a distal end and constituted by a plurality of wire filaments extending from the proximal end towards the distal end, comprising::
selecting at least one wire;
weaving the basket from the wire; and
forming a shape of the retrieval basket, thereby imparting structural rigidity and dilatation abilities to the basket when opened,
the method characterized in that said weaving includes forming portions from wire filaments that are bound together to define a plurality of strands, said plurality of strands ramify at corresponding branching points into loops having various shapes and sizes, at least a part of the loops are overlapped and/or interlaced so as to define a net at least at the distal end.

In accordance with another one broad aspect of the invention, there is provided a retrieval basket for entrapping and retaining an object located in a body for its extraction therefrom, the basket comprising a structure having a proximal end and a distal end and constituted by a plurality of filaments extending from the proximal end towards the distal end, said filaments are made of a material that imparts structural rigidity and dilatation abilities to the basket when opened,
characterized in that said filaments have portions that are bound together to define a plurality of strands at the proximal end, said plurality of strands ramify at corresponding branching points into loops having various shapes and sizes, at least a part of the loops are overlapped and/or interlaced so as to define a net at least at the distal end, thereby imparting structural rigidity and dilatation abilities to the basket when opened.

In accordance with further broad aspect of the invention, there is provided a surgical device for removing a foreign object from a body, comprising:
a retrieval basket for entrapping and retaining an object located in a body for its extraction therefrom, the basket comprising a structure having a proximal end and a distal end and constituted by a plurality of filaments extending from the proximal end towards the distal end; at least a part of the filaments are overlapped and/or interlaced so as to define a net at least at the distal end, and thereby imparting structural rigidity and dilatation abilities to the basket when opened; and
a basket control assembly coupled to the basket, comprising a tubular sheath adapted to penetrate into the body for reaching the object, a manipulator for manipulating the basket for extraction the object from the body, and a manipulation cable arranged within the sheath for connecting the basket to the manipulator, where the assembly is configured for retracting the basket within the sheath and protracting the basket therefrom for its opening.

In accordance with yet another one broad aspect of the invention, there is provided a surgical device for removing a foreign object from a body, comprising:
a retrieval basket constituted by a plurality of filaments for entrapping and retaining an object located in a body for its extraction therefrom; and
a basket control assembly coupled to the basket, comprising a tubular sheath adapted to penetrate into the body for reaching the object, a manipulator for manipulating the basket for extraction the object from the body, and a manipulation cable arranged within the sheath for connecting the basket to the manipulator, wherein said manipulation cable is constituted by said plurality of filaments extending from the basket towards the manipulator, the assembly is configured for retracting the basket within the sheath and protracting the basket therefrom for its opening.

In accordance with still another one broad aspect of the invention, there is provided a surgical device for removing a foreign object from a body, comprising:
a retrieval basket constituted by a plurality of filaments for entrapping and retaining an object located in a body for its extraction therefrom; and
a basket control assembly coupled to the basket, comprising a tubular sheath adapted to penetrate into the body for reaching the object, a manipulator for manipulating the basket for extraction the object from the body, and a manipulation cable arranged within the sheath for connecting the basket to the manipulator, wherein said manipulation cable is configured as a separate unit selected from a rod, cable and wire, the assembly is configured for retracting the basket within the sheath and protracting the basket therefrom for its opening.

There has thus been outlined, rather broadly, the more important features of the invention in order that the detailed description thereof that follows hereinafter may be better understood. Additional details and advantages of the invention will be set forth in the detailed description, and in part will be appreciated from the description, or may be learned by practice of the invention.

### BRIEF DESCRIPTION OF THE DRAWINGS

In order to understand the invention and to see how it may be carried out in practice, a preferred embodiment will now be described, by way of non-limiting example only, with reference to the accompanying drawings, in which:
Fig. 1 is a schematic view, partially in a longitudinal cross-section, of the distal end of a conventional surgical extractor;
Fig. 2A-Fig. 2D shows a plan and top view of four examples of the retrieval basket manufactured which are not in accordance to the present invention;
Fig. 2E shows a plan and top view of a retrieval basket in accordance to the present invention;
Fig. 3 is a flowchart diagram that describes the method for manufacturing a retrieval basket from a single wire piece, in accordance with one embodiment of this invention;
Fig. 4 is a flowchart diagram that describes the method for manufacturing a retrieval basket from several wires, in accordance with another embodiment of this invention;
Figs. 5A-5C are illustrations of the steps of the method for manufacturing a retrieval basket, in accordance with one embodiment of the invention;
Fig. 6 is an example of the basket obtained by the process of Fig. 3;
Fig. 7 is a schematic view of a surgical device for extraction of an object from a body, according to one embodiment, but equipped with a basket which is not subj ect of the invention;
Fig. 8 is a schematic view of a surgical device for extraction of an object from a body, according to another embodiment but equipped with a basket which is not subject of the invention;
Fig. 9 is a schematic view of a surgical device for extraction of an object from a body, according to yet another embodiment but equipped with a basket which is not subject of the invention; and
Figs. 10A-10F illustrate schematically examples of how distal ends of the loops are intertwined; and.
Figs. 11A-11B are illustrations of the method for manufacturing a retrieval basket, in accordance with a further embodiment of the invention.

### DETAILED DESCRIPTION OF SPECIFIC EMBODIMENTS

The principles of the method for surgical device according to the present invention may be better understood with reference to the drawings and the accompanying description, wherein like reference numerals have been used throughout to designate identical elements. It being understood that these drawings are given for illustrative purposes only and are not meant to be limiting.

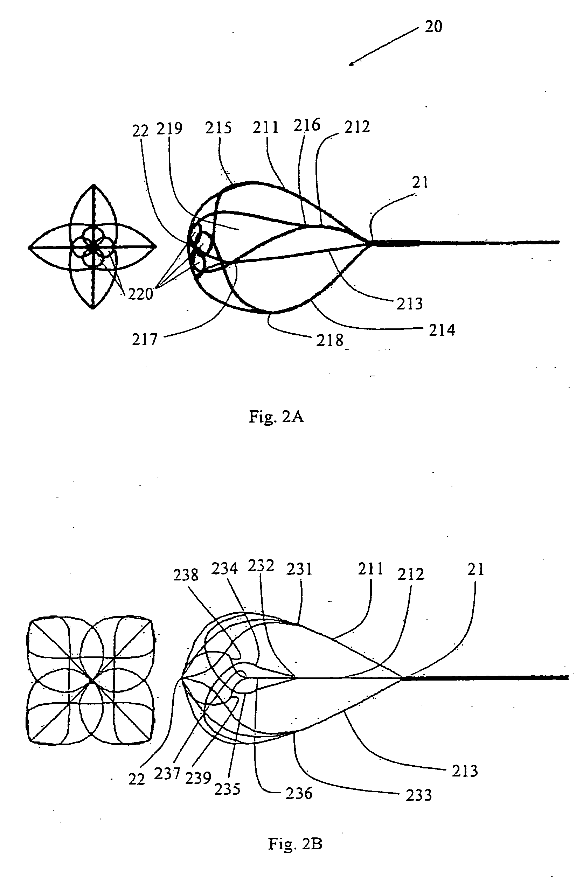

Referring now to Fig. 2A through Fig. 2D, four examples of a retrieval basket 20 shaped in a parachute-like fashion are illustrated, which provide helpful information for understanding this invention but are not subject of the present invention. In general, the basket 20 comprises a structure having a proximal end **21** and a distal end **22.** The structure is formed by a plurality of filaments fabricated from a single wire in the manner describe below. The filaments extend between the proximal end **21** and the distal end **22.** At least a part of the filaments, which originate from the proximal end **21,** can arrive at the proximal end **22** after winding. The filaments are entwined and spatially arranged in such manners that upon protracting from a sheath they can be readily spread out. In the vicinity of the proximal end **21** the filaments form strands **211, 212, 213** and **214.** These strands ramify at branching points **215, 216, 217,** and **218**, into loops with various shapes and sizes.

According to the example shown in **Fig. 2A****,** each strand includes two entwined wire filaments that form loops after the branching point. For instance, after the ramification of the strand **216** the loop **219** emerges from the branching point **216.** As can be seen, the loops emerged from the branching points are interlaced so as to define a net at the distal end. Furthermore, the loops are formed in such a manner that additional small loops **220** are arranged at the distal end **22**. The loops provided thereby overlap and interlace, thus defining the dense net-like structure of the basket. It should be appreciated that the size of holes (cells) of the net decreases from the proximal end towards the distal end. In other words, the density of the net increases from the proximal end to the distal end of the structure. The increased density of the net allows for entrapment and retention of small objects which would not ordinary be retained in the aforementioned conventional retrieval baskets having the relatively wide spacing (**16 in** **Fig. 1**).

It should be appreciated that in contrast to the conventional baskets (e.g., the basket shown in **Fig. 1**), the filaments of the basket of the present invention are overlapped and interlaced so as to define a net, at least in the vicinity of the distal end. This feature imparts an enhanced structural rigidity and dilatation ability to the basket when it is opened. By providing the loops formed by the filaments with various shapes and sizes it is possible to vary also the size and shape of the cells, formed at the intersection of the loops, thus control the capture and retention abilities of the basket.

In the example shown in **Fig. 2B** only three strands **211, 212** and **213** of the retrieval basket **20** can be seen. Each strand is formed of four entwined wire filaments. These strands ramify at branching points **231, 232** and **233.** After the ramification the strands form interlaced loops. For example, the strand **212** ramifies at the branching point **232** into filaments **234, 235** and a strand **236.** The filaments **234** and **235** form two corresponding loops. The strand **236** consisting of two remaining entwined filaments ramifies at a branching point **237** and provides a loop formed of the filaments **238** and **239.** The loops formed thereby are interlaced so as to form a net structure.

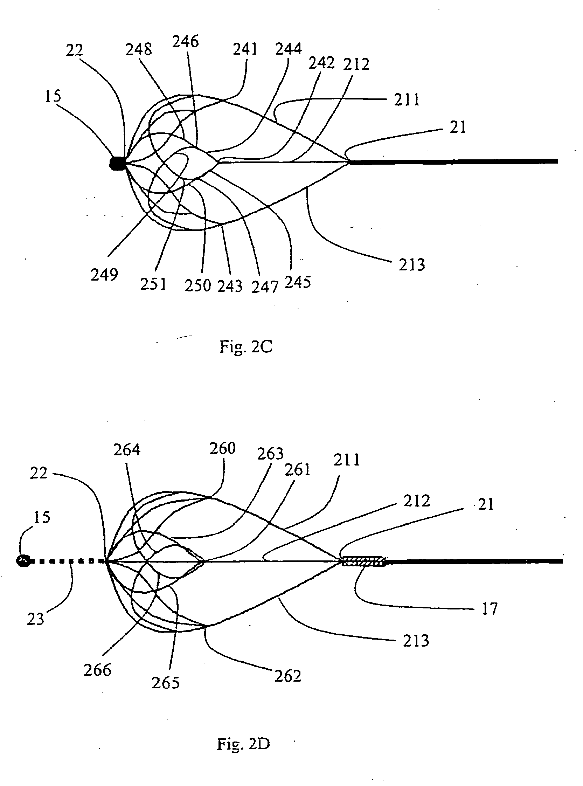

Referring now to **Fig. 2C****,** yet another example of the retrieval basket **20** is illustrated. According to this example, the retrieval basket **20** includes four strands configured in branches. Only three such strands **211, 212** and **213** can be seen in **Fig. 2C****.** Each strand is formed of four entwined wire filaments. The **211, 212** and **213** ramify at branching points **241, 242, 243** and result in interlaced loops. For example, the strand **212** ramifies at the branching point **242** into two branches **244** and **245,** consisting of two entwined filaments. In turn, the branches **244** and **245** ramify at branching points **246** and **247,** and provide loops formed of filaments **248, 249, 250** and **251,** respectively. The loops formed thereby are interlaced so as to form a net structure.

Referring now to **Fig. 2D****,** still a further example of the retrieval basket **20** is illustrated. According to this example, the retrieval basket **20** includes four strands configured in branches. Only three such strands **212, 213** and **214** can be seen in **Fig. 2D****.** Each strand is formed of four entwined wire filaments. These strands ramify at branching points **260, 261, 262** and result in interlaced loops. For example, the strand **212** ramifies at the branching point **261** into the loops formed of filaments **263, 264, 265** and **266.** The loops formed thereby are interlaced so as to form a net structure. According to this embodiment of the invention, the strands of the basket are bound together at the proximal end **21** by means of the connector **17.**

It is apparent that the retrieval basket of the present invention is not bound to the examples of the tipless baskets shown in **Fig. 2A** and **Fig. 2B**. If necessary, the basket may be constructed to have a tip at the distal end of the basket **22**, for example, in by means of a bushing (**15** in **Fig. 2C** and **Fig. 2D**), with the help of which the wire filaments are bound together. When desired, the tip **15** can be arranged at the distal end of a guiding rod (shown by dashed line **23** in **Fig. 2D**). The guiding rod **23** can, for example, be formed from intertwined wire filaments. The guiding rod **23** can function as a guide to facilitate the penetration and movement of the basket within the body organs.

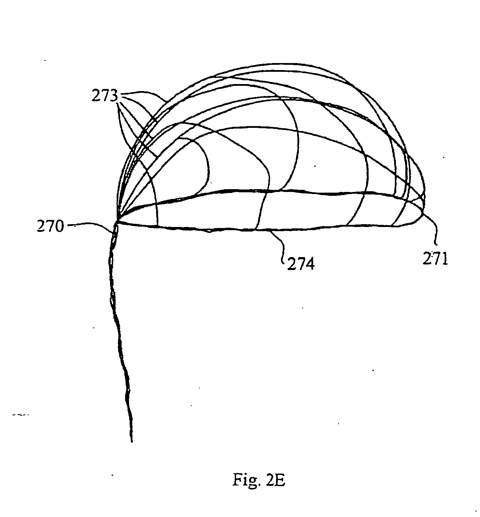

Referring now to **Fig. 2E**, still a further example of the retrieval basket **20** of the invention is illustrated. According to this example, the retrieval basket **20** comprises a spoon-like structure formed of a plurality of wire filaments. The basket **20** has a proximal end **270** and a distal end **271**. In a region of the structure at the proximal end, the filaments are bound and form a plurality of strands **273**. The filaments extend from the proximal end towards the distal end and are configured in the form of interlaced loops so as to form a net structure. The filaments, which originate from the proximal end, arrive at the proximal end after winding. According to this embodiment, one strand (a strand **274**) of the plurality of strands **273** is common for all the filaments.

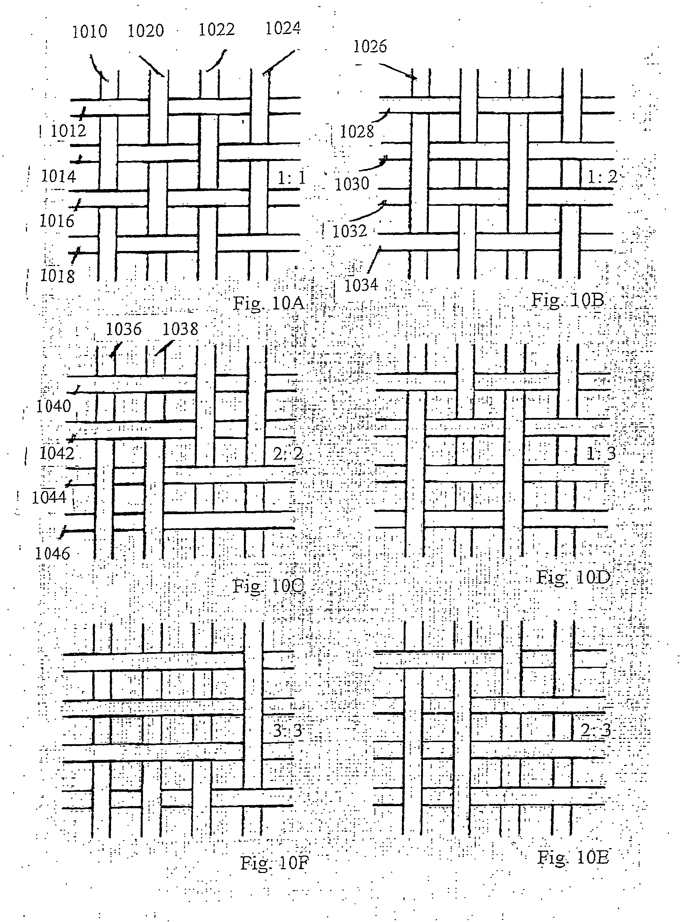

Referring now to **Figs. 10A-10F**, it will be explained how individual wire filaments, from which the loops are made, could overlap and/or interlace. Note that the term "overlap" herein is assigned to such arrangement of the filaments, in which one element crosses another filaments, i.e., one of the filaments always being over or under the other filaments. The term "interlace" herein is assigned to the situation when at least one filament interweaves with the other filaments, i.e., one of the filaments passes first above the crossed filament and then passes under the next crossed filament.

In **Figs. 10A-10F** different patterns corresponding to a possible arrangement of individual filaments defining the cells are shown schematically and with exaggeration. For the sake of simplicity, the wire filaments are depicted as vertical and horizontal bands overlapping at a right angle and defining orthogonal pattern, consisting of four vertical and four horizontal bands. However, it should be understood that in reality, thin wire filaments form the cells. The filaments are directed with respect to each other not necessarily at a right angle, and their amount is not limited to a four by four pattern.

**Fig. 10A** shows a pattern in which all wire filaments interlace one with another, i.e., each horizontal wire filament interlaces with all vertical filaments and vice versa. It is seen, for example, that a vertical filament **1010** goes first under a horizontal filament-**1012**, then above a horizontal filament **1014,** then again under a horizontal filament **1016** and finally again above a horizontal filament **1018.** On the other hand the horizontal filament **1012** goes first above the vertical filament **1010,** then under a vertical filament **1020.** then again above a vertical filament **1022** and then again under a vertical filament **1024.** The rest of filaments are arranged similarly.

**Fig. 10B** depicts another situation, in which the filaments both interlace and overlap with intersection. It is seen that a vertical filament **1026** interlaces with horizontal filaments **1028, 1030, 1034** and overlaps with the perpendicularly directed filament **1032.**

In **Fig. 10C** is shown still a new pattern, consisting of overlapped and interlaced filaments. It is seen for this example that two neighboring vertical filaments **1036, 1038** go under two neighboring horizontal filaments **1040, 1042** and then go above two next horizontal filaments **1044, 1046.**

**Figs. 10D-10F** show further possible patterns, consisting of overlapped and interlaced filaments. In particular, in the pattern shown in **Fig. 10F****,** each filament overlaps with three perpendicular filaments and interlaces with only one filament.

It can be appreciated that the patterns depicted in **Figs. 10A** and **Fig. 10F** represent two extreme situations, corresponding respectively to the pattern in which all filaments are interlaced and to the pattern in which only one filament is interlaced, while the other filaments are overlapped.

Having explained various cells patterns, in which the filaments of the loops might be arranged, hereinbelow, a process of manufacturing the retrieval basket 20 (illustrated in **Fig. 2A** through **Fig. 2F**) will be described.

The process begins with a step of selection of a predetermined number of wires having a predetermined diameter and length.

According to one non-limiting example of the process, the manufacturing of the retrieval basket is carried out from one piece of wire.

According to another non-limiting example of the process the manufacturing of the retrieval basket is carried out from several wires.

It should be noted that the wires selected for the construction of the basket can be single-filament wires, or when desired, can be multi-filament wires.

Furthermore, the process for the fabrication of the retrieval basket includes weaving the retrieval basket from one piece of wire or from several wires.

For manufacturing a retrieval basket, according to the invention, at least a part of the weaving can be performed on a weaving jig. For this purpose, the jig can have a predetermined pattern formed by grooves configured on the jig's surface.

The manufacturing process also includes forming a shape of the retrieval basket that includes defining the basket's shape and topology (pattern). According to one embodiment of the invention, for the defining of the shape and topology at least a part of the filaments are bound on a shape-forming jig, which for this purpose has a predetermined shape.

The forming of the shape can further include shape storing annealing the basket mounted on the shape-forming jig. Such forming a shape allows the basket to impart the structural rigidity and dilatation abilities, when the basket is opened.

It should be appreciated that the weaving jig and the shape-forming jig can be the same unit or two different units, each having its own functions.

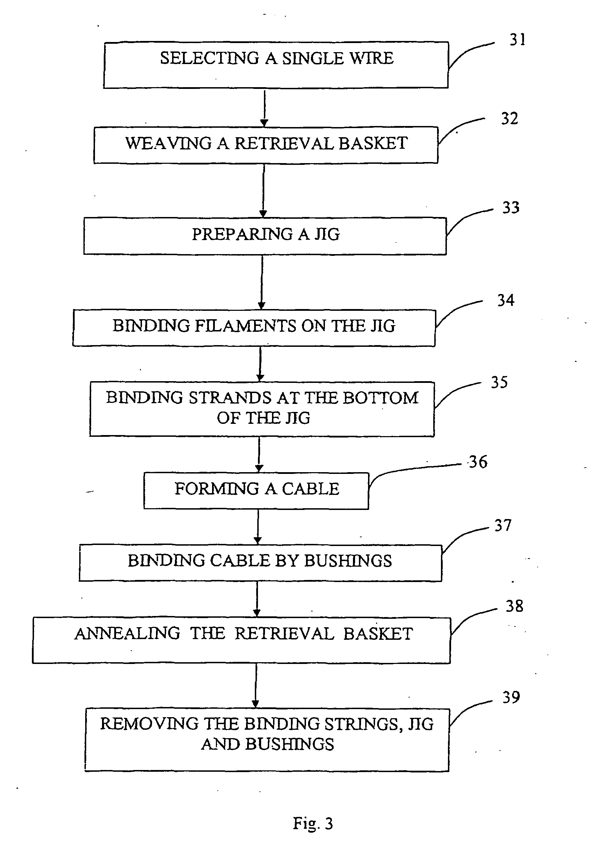

Referring now to **Fig. 3**, an example of the process for the fabrication of the retrieval basket **20** from a single piece of wire is described in detail. The process begins with a step **31** of selection of a single wire having a predetermined diameter and length. It should be appreciated that this length depends on the shape, pattern and size of the basket **20**. For example, for a basket having the size of 3 Fr, the minimal diameter and length of the wire selected for the retrieval basket 20 is about 0.1 mm Fr and 100 cm, respectively.

Typically, the wire utilized for the fabrication of the retrieval basket **20** is made of a suitable shape memory or elastic material. According to one embodiment of the invention, the wire is made of metallic material. The metallic material can be selected from NiTi based alloy (e.g., Nitinol) and stainless steel.

According to another embodiment of the invention, the wire is made of non-metallic material, e.g. Capron.

According to a still further embodiment of the invention, the filaments of the basket are covered by an insulating layer. The insulating layer can, for example, be made of Teflon. The advantage of Teflon is its thermal resistance and low coefficient of mechanical friction, which leads to an additional reduction of traumatism.

According to yet another embodiment of the invention, the wire is made of a material that has thermo-mechanical shape memory characteristics.

As was noted above, the wire selected for the construction of the basket can be a single-filament wire, or, when desired, can be a multi-filament wire.

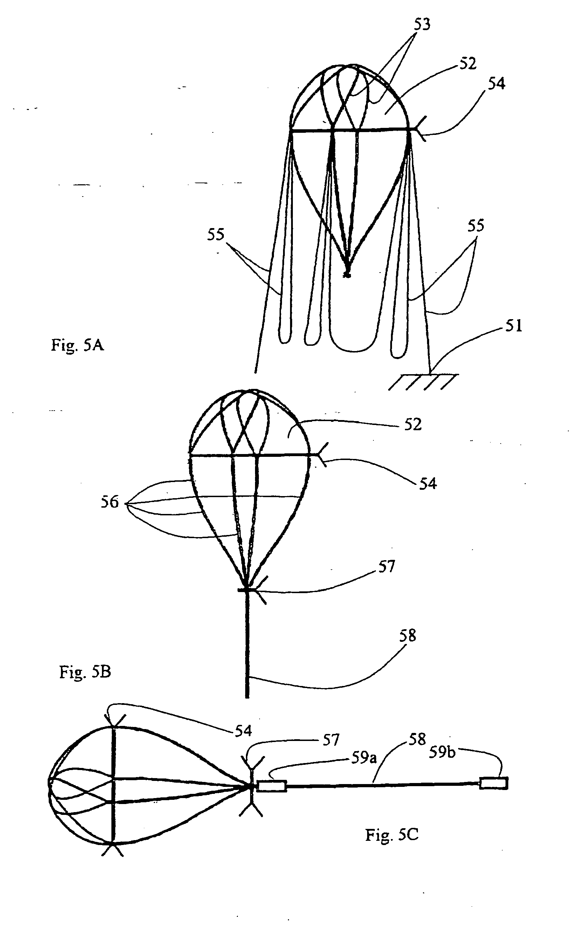

Referring to **Fig. 3** and **Figs. 5A****-5C** together, the process for the fabrication of the retrieval basket 20 further includes a step **32** of weaving the retrieval basket from the selected single wire, according to one embodiment of the invention. According to this embodiment, the process includes a step **33** of preparing a jig **52** designed for weaving and shaping the basket **20.** The jig **52** can have a structure including grooves arranged in accordance with the desired pattern of the basket **20** and can have a shape that imitates the desired shape of the basket **20.**

According to this embodiment, one end **51** of the single wire piece is fixed, while the other end is put on the top part of the jig **52** and moved along the grooves in accordance with a desired pattern of the weaving and topology of the basket structure. The weaving of the basket continues by moving the free end away from the jig and returning it thereto by overlapping and/or interlacing the wire filaments, as long as required to form a net.

In order to avoid the slipping down of the wire from the jig and unweaving of the basket, filaments **53** woven thereby are tied up (step **34)** on the jig **52** by a first string **54** worn round the jig, as shown in **Fig. 5A****.** An example of the first string includes, but is not limited to, a copper wire having strength sufficient to maintain the fixture of the basket on the jig.

Further, the fixed end (**51** in **Fig. 5A**) of the wire is released, and filaments **55** dangled from the jig are bound together to form strands **56,** as shown in **Fig. 5B****.** The strands **56** are in turn tied up by a second string **57** at the bottom part of the jig **52** for defining a shape of the basket (step **35**). An example of the second string includes, but is not limited to, a copper wire having sufficient strength to maintain the strands **56** together. The strands **56** are then bound together (step **36**) to form a cable **58.**

Thereafter, two temporary bushings **59a** and **59b** are put on the ends of the cable **58** (step **37**). The bushings **59a** and **59b** can, for example, be in the form of pipes made of metal, e.g., Ni, stainless steel, etc. The bushings **59a** and **59b** are then squeezed (crimped) for reducing their inner diameter, and thus fixation of the filaments therein. It should be appreciated that this is only a non-limiting example of the filaments' fixation. Other ways can also be used, e.g., soldering, welding or gluing. When desired, the ends of the bushings **59a** and **59b** (for fixation on the cable **58**) can be further additionally treated by soldering, welding and/or polishing.

Specifically, for a metallic basket having the size of 3 Fr, according to the example described above, the bushings can have the inner diameter of 0.7 mm and the length of 4 mm. After squeezing, the inner diameter of the bushings **59a** and **59b** put on the cable **58,** can, for example, achieve a value of 0.6 mm. It should be understood that these values can be varied depending on the wire's material, construction, etc.

The process of **Fig. 3** further includes a step **38** of shape storing annealing that depends on the materials of the wire. For example, when such material is Nitinol, the annealing is carried out at the temperature of about 450 °C - 600 °C over at least about 10 min. It should be appreciated that such heat treatments could relieve internal stresses in the material and provide the memorization of the basket shape.

It should be appreciated that the invention is not limited to the specific implementation of the annealing step. For example, the annealing can be carried out in an oven configured for this purpose. However, when the wire is selected from a conductive material, the annealing can be carried out by passing electric current through the filaments of the basket.

After the annealing, the jig **52,** the first string **54,** the second string **57** and the temporary bushings **59a** and **59b** are removed (step **39**).

It should be appreciated that the process of manufacturing the retrieval basket is not limited to forming the basket from one piece of wire. According to another example, the retrieval basket can also be formed from several wires.

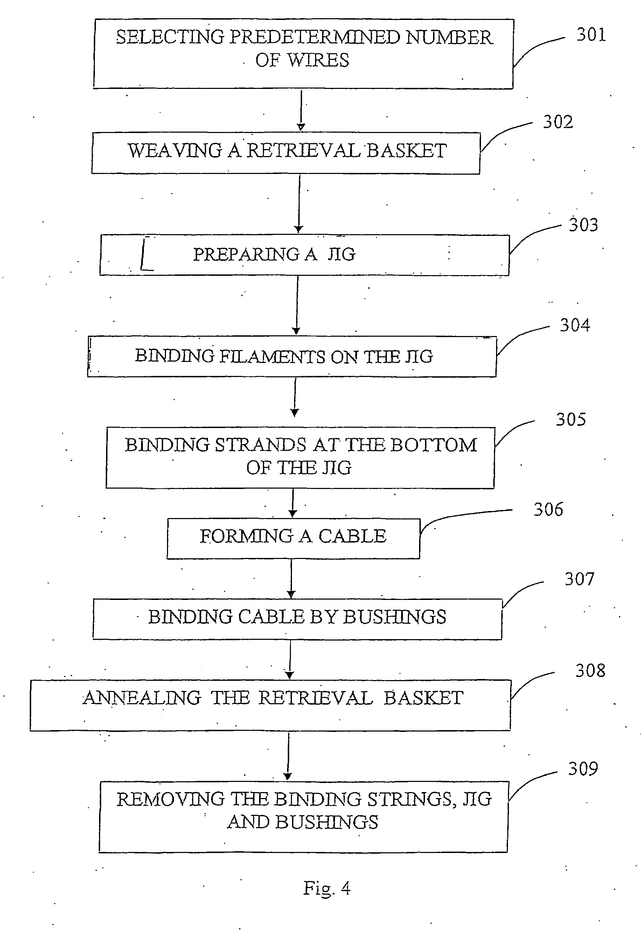

Referring now to **Fig. 4****,** a further example of the process for fabrication of the retrieval basket **20** from several wires is shown in detail. The process begins with a step **301** of selection of a predetermined number of wires having a predetermined diameter and length. It should be appreciated that the choice of the wire parameters depends on the shape, pattern and size of the basket. For example, for a basket shown in **Fig. 2A** and having the size of 3 Fr, the number of the wires is 8, the minimal diameter and length of the wires are 0.12 mm and 100 cm, respectively.

The process for fabrication of the retrieval basket includes a step **302** of weaving the retrieval basket from the selected number of wires.

According to one example, the process includes a step **303** of preparing a jig **41** designed for weaving the basket **20.** In particular, the jig **52** suitable for the process of fabrication of the basket from a single piece of wire is also suitable for the process shown in **Fig. 4****.**

For purpose of this embodiment of the process shown in **Fig. 4****,** firstly, a wire is selected. One end of this wire is moved along the grooves of the jig in accordance with a desired pattern of the weaving. When this wire is dropped into the grooves, the free ends of the wires are placed at the bottom of the jig. Thereafter, another wire is taken, and the process continues with this wire. As a result of such weaving, the wire filaments are intercrossed and/or interlaced in accordance with the desired topology of the basket structure.

The further steps of the process shown in **Fig. 4** for forming the basket from several wires are similar to the process steps **34** through **39** of **Fig. 3A.** Accordingly, the process further includes the steps of binding the wire filaments on the jig (step **304**) by a first string; forming strands and the binding thereof at the bottom of the jig (step **305**) by a second string; bounding the strands and forming a cable (step **306**); binding the cable by bushings (step **307**); annealing the basket (step **308**); and removing the jig along with the binding strings and bushings (step **309**).

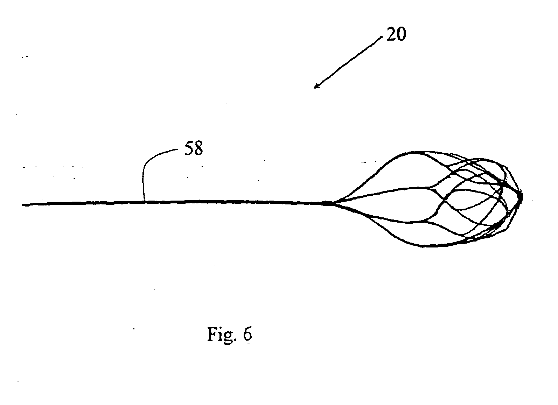

Accordingly, **Fig. 6** shows an example of the basket obtained by the process shown in **Fig. 3** or **Fig. 4****.**

It should be appreciated that when required, the process for the fabrication of the retrieval basket can include a step of binding the filaments at the distal end **22** together to form a basket tip. Such binding can, for example, be done by a bushing. When desired, after the tip, the wire filaments can be expended to form a guiding rod (shown by dashed line **23** in **Fig. 2D**).

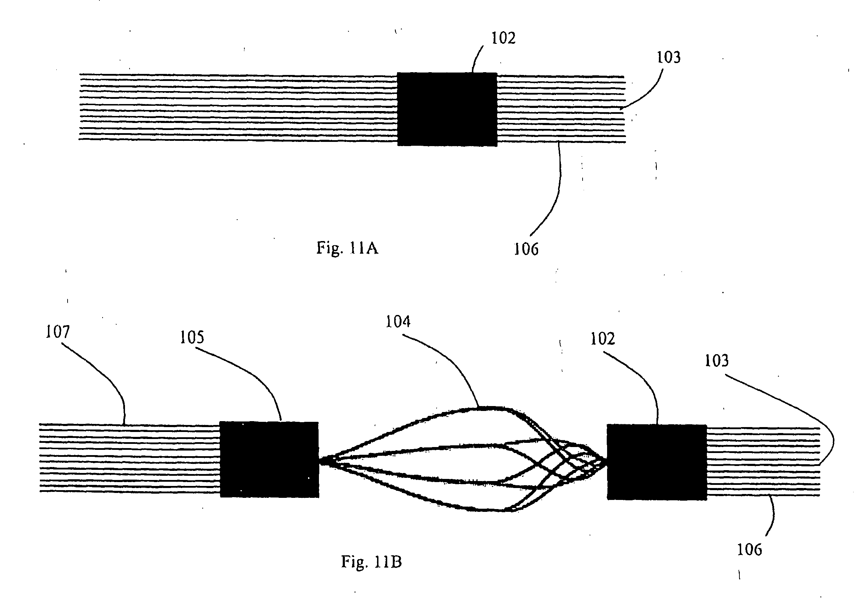

Referring to **Figs. 11A** and **11B** together, a. still further example of the process for fabrication of the retrieval basket **20** from several wires is shown in detail.

The process starts with a step of selection of a predetermined number of wires having a predetermined diameter and length. It should be appreciated that the choice of the parameters of the wires depends on the shape, pattern and size of the basket.

Furthermore, the process for fabrication of the retrieval basket includes a step of weaving the retrieval basket from the selected number of wires. For this purpose, the wires are joined together in a bunch **101,** and then, a bushing **102** is fixed on the bunch 101 at a predetermined distance from an end 103 of the bunch 101. A section 106 of the bunch 101 between the bushing 102 and the end 103 is devised for forming a guide rod. Therefore, the length of this distance depends on the desired length of the guide rod. The basket is woven by overlapping and/or interlacing the wire filaments so that to form a net 104. After the weaving, another bushing 105 is fixed on the bunch 101, in order to avoid the unweaving of the basket.

Thereafter, the process for fabrication of the retrieval basket includes a step of forming a basket's shape. For this purpose, the basket is mounted, aligned and fixed on a shape-forming jig (not shown), which has a predetermined shape. Further, the step of forming a basket's shape includes shape storing annealing. For example, when the wires are selected from Nitinol, the annealing can be carried out at the temperature of about 400 °C - 600 °C over at least about 10 min.

According to one embodiment of the invention, the heating at such temperatures can be performed in an oven. According to another embodiment of the invention, the heating at such temperatures can be performed by passing an electric current through the wire filaments.

Furthermore, when required, the process for fabrication of the retrieval basket can include a step of forming a guide rod. For this purpose, a certain number of superfluous wire filaments in the section 106 are cut off, and the remaining wire filaments are twisted together. Preferably, the twisted wire filaments are then squeezed and heated. The heating can, for example, be performed by applying a voltage (e.g., about 4 V over about 2-3 sec) across the section 106.

The process for fabrication of the retrieval basket can include a step of bounding the wire filaments of a section 107 and forming a cable (that is similar to the cable 58 in Figs 5B-5C). The forming of the cable can, for example, be performed by twisting and squeezing the filaments and applying a voltage (e.g., about 20-30 V over about 3-10 sec) across the section 107.

Those skilled in the art will now recognize the substantial difference between the prior art retrieval baskets of Fig. 1, Figs. 2A-2D and basket 2E produced by the novel process of the present invention. In particular, according to the prior art methods, the baskets are assembled from a number of wires having the predetermined configuration along a basket axis with the wire ends binding together at the proximal and distal ends. In contrast to the prior art techniques, the novel process of the present invention is based on weaving the basket such that the wire loops formed as a result of the weaving of the filaments are interlaced so as to define a net, and thereby to impart an enhanced structural rigidity and dilatation ability to the basket.

From the foregoing description it should be appreciated that retrieval baskets constructed in accordance with the present invention can comprise a variety of user desired shapes, number of wires, topology of weaving, and filament spacing.

The basket manufactured from a single wire or several wires as described above, can be used in the production of a surgical device for extracting objects from a body during the treatment of the biliary or urinary systems. Such treatment may include the extraction of stones, e.g., gall stones, kidney stones, etc.

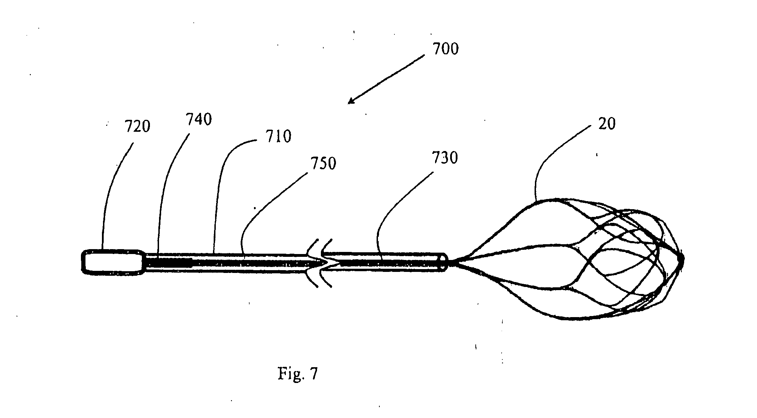

Referring now to Fig. 7, a schematic view of a surgical device 700 for extraction of an object from a body is shown, but equipped with a basket which is not according to one embodiment of the invention. The device 700 includes the retrieval basket 20 obtained by the method described above, a flexible tubular catheter 710, configured as a sheath, adapted to penetrate along the body passages near the location of the object (not shown), and a manipulator 720 suitable for manipulating the retrieval basket 20,

It can be appreciated by a person versed in the art that in contrast to the conventional extractor (shown in Fig. 1), the device of the present invention depicted in Fig. 2A through Fig. 2C does not include the convector (17 in Fig. 1). Additionally, a manipulation cable 730 located within the catheter 710 includes the cable 58 that is formed as an integral part of the basket 20. In other words, the manipulation cable 730 is not a separate element (cable 12 in Fig. 1). It can be appreciated that when the cable 58 itself is used as the manipulation cable, then it is directly connected to the manipulator 720. Such connection can be performed, for example, by soldering. However, when the length of the cable 58 is not sufficient for manipulating the surgical device, the manipulation cable 730 can include the cable 58 and a pushing element 740 (e.g., rod, cable or wire), also arranged within the catheter. In this case, the pushing element 740 is connected to the manipulator 720 (at one end) and the cable 58 (at another end), thereby extending the manipulation cable length.

In practice, a surgeon can manipulate the cable or pushing element by means of the manipulator 720, and thus the basket 20 can be either retracted within the catheter 710 or protracted therefrom. The surgeon, by holding the manipulator 720, can also maneuver the catheter 710 within the body organ (not shown), (e.g. to displace it by turning, pushing or pulling),

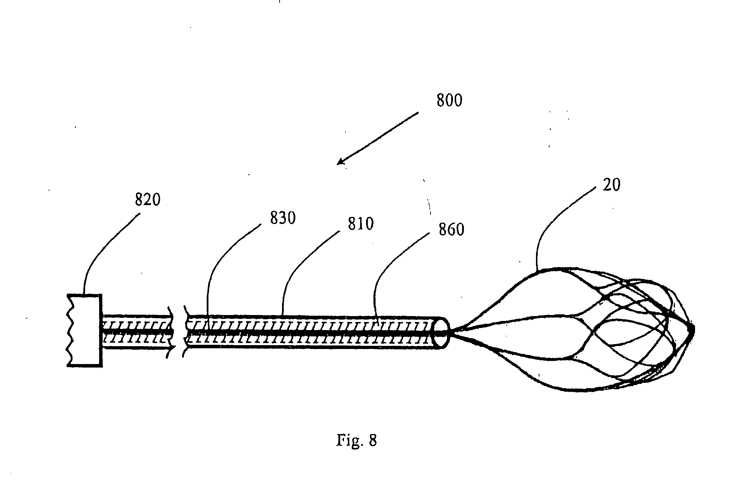

Referring to Fig. 8, a schematic view of a surgical device 800 for extraction of an object from a body is shown, but equipped with a basket which is not according to an embodiment of the invention.

This embodiment is devised for overcoming the aforementioned drawback of the conventional surgical extractor (10 in Fig, 1) and inherent also to the surgical device (700 in Fig. 7), which is associated with the discrepancy of the inner diameter of the catheter and the diameter of the manipulation cable. As can be appreciated by a person versed in the art, due to this discrepancy, an air gap (18 in Fig. 1 and 750 in Fig. 7) between the inner wall of the catheter and the manipulation cable significantly affects the deformation properties of the catheter and the ability of the catheter to bend without destruction on large angles.

The device 800 includes the retrieval basket 20 obtained by the method described above, a flexible tubular catheter 810, configured as a sheath, a manipulator 820, manipulation cable 830 located within the catheter 810, and a filling tubing 860. According to this embodiment of the invention, the filling tubing 860 is put on a major portion of the manipulation cable 830 that is formed from the cable 58.

The outer diameter of the filling tubing 860 should have a sufficient magnitude to substantially fill the gap between the inner wall of the catheter 810 and the manipulation cable 830, in order to enhance the ability of the catheter to bend without destruction on large angles, while enabling for the manipulation cable 830 to move without significant friction within the catheter.

According to one embodiment of the invention, the filling tubing 860 is formed of a heat-shrinking material. An examples of the heat-shrinking tubings includes, but are not limited to, PTFE heat-shrinking tubing SLW-HS NATURAL available from ZEUS.

A process for preparing the surgical device 800 includes putting the filling tubing 860 on the cable (58 in Fig. 6) of the basket 20 manufactured according to the method described above, and heating the assembly obtained thereby at the temperature of 80 °C - 120 °C over at least about 1 min.

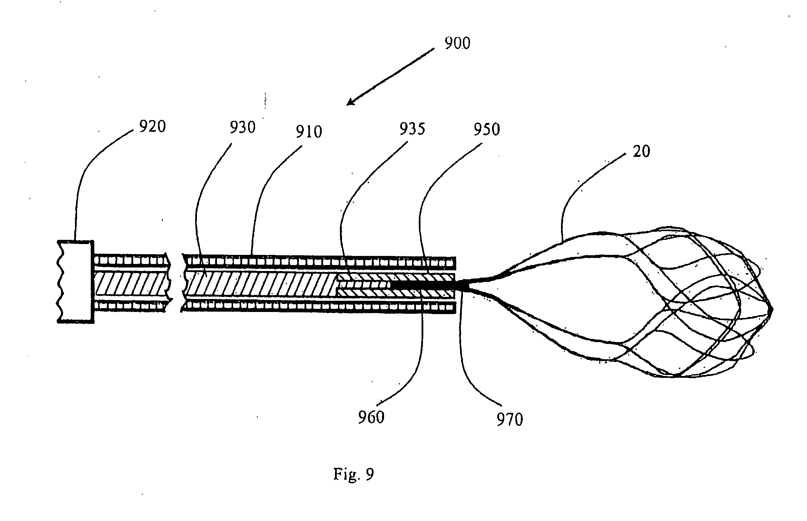

Referring to Fig. 9, a schematic view of a surgical device 900 for extraction of an object from a body is shown, but equipped with a basket which is not according to an embodiment of the invention. The device 900 includes the retrieval basket 20 obtained by the method described above, a flexible tubular catheter 910, configured as a sheath, a manipulator 920, and a manipulation cable 930 located within the catheter 910.

According to this embodiment, the manipulation cable 930 is produced as a separate unit. In this case it is coupled to the manipulator 920 at a cable proximal end 934. and to a common basket strand 960 at a cable distal end 935. The joint between the common strand 960 and the cable distal end 935 is accomplished by means of a bushing 950. The common strand 960 is a portion of the cable 58 that is cut in the vicinity of an original branching point 970 of the basket 20. The bushing 950 can, for example, be in the form of a pipe made of Ni, stainless steel, etc.

In order to enhance the ability of the catheter to bend without destruction on large angles the diameter of the major portion of the manipulation cable 930 has a magnitude sufficient to fill substantially the gap between the inner wall of the cable **930** at the distal end **935** is different from its major diameter and has a magnitude substantially close to the magnitude of the common basket strand **960.**

In order to obtain a joint between the manipulation cable **930** and the common basket strand **960** the process for preparing the surgical device **900** includes providing the bushing **950;** putting the bushing **950** on a portion of the common basket strand **960** and on the distal end **935**; and squeezing the bushing **950** for reduction of its inner diameter to the magnitude sufficient to maintain the joint. According to one embodiment, the magnitude of the diameter of the bushing after such squeezing is close to a magnitude of the major diameter of the manipulation cable.

As such, those skilled in the art to which the present invention pertains, can appreciate that while the present invention has been described in terms of preferred embodiments, the concept upon which this disclosure is based may readily be utilized as a basis for the designing of other structures and processes for carrying out the several purposes of the present invention.

It should be understood that the device of the present invention is not limited to the urological treatment of a human body. It can be successfully employed for surgical treatments of animals as well. Furthermore, the present invention is not limited strictly to fabrication of a surgical device for extracting calculi during the urological treatment. Such a device is suitable for other surgical treatments, which might require retrieval of foreign objects from the body systems, e.g. from blood vessels etc.

Moreover, the present invention is not limited to fabrication of medical devices, and the extractor device can be used to extract any type of article from a wide range of inaccessible locations such as inside a pipe or tube (for example, the waste outlet of a domestic sink) or inside a chamber within a large piece of machinery which would be difficult to dismantle.

In the method claims that follow, alphabetic characters used to designate claim steps are provided for convenience only and do not imply any particular order of performing the steps.

Also, it is to be understood that the phraseology and terminology employed herein are for the purpose of description and should not be regarded as limiting.

It is important, therefore, that the scope of the invention is not construed as being limited by the illustrative embodiments set forth herein. Other variations are possible within the scope of the present invention as defined in the appended claims and their equivalents.

## Claims

1. A method for manufacturing a retrieval basket having a proximal end (270) and a distal end (271) and constituted by a plurality of wire filaments extending from the proximal end towards the distal end, comprising:
selecting at least one wire;
weaving the basket from the wire, said weaving includes forming portions that are bound together to define a plurality of strands (273 and 274) at the proximal end (270) each strand including a plurality of the filaments, said plurality of strands (273 and 274) ramify at corresponding branching points into loops having various shapes and sizes, at least a part of said loops are overlapped and/or interlaced so as to define a net at least at the distal end (271); and
forming a shape of the retrieval basket, thereby imparting structural rigidity and dilatation abilities to the basket when opened;
**characterized in that** said weaving is such that one of the strands (274) of said plurality of strands (273 and 274) is common for all the wire filaments and forms a loop, and **in that** said retrieval basket is formed in a spoon-like shape,

2. The method of claim 1 further including binding the strands together at said proximal end for forming a manipulation cable (58).

3. The method of claim 2 further including binding the filaments, of the manipulation cable (58) by temporary bushings (59a, 59b).

4. The method of claim 3 further including annealing said retrieval basket.

5. The method of claim 1 including binding the filaments at the distal end (271) together for forming a basket tip.

6. The method of claim 1 wherein said forming of the shape includes preparing a shape-forming jig (52) having a predetermined shape.

7. The method of claim 1 wherein said weaving includes preparing a weaving jig (52) having a predetermined a pattern formed by grooves configured on a surface of the jig.

8. The method of claim 7 wherein at least a part of the weaving of the basket is performed on said weaving jig (52), wherein said weaving includes:
fixing one end of the wire, while the other end is putting on the top part of the jig; and
moving the free end along the grooves by taking it away from the jig and returning it thereto together with overlapping and/or interlacing the wire filaments, in accordance with a desired pattern of the weaving and topology of the basket structure.

9. The method of claim 6 wherein said forming of the shape includes blinding the filaments and the strands on said shape-fomning jig (52).

10. The method of claim 1 wherein said weaving of the basket includes:
selecting a predetermined number of wires;
joining the wires together in a bunch (101);
fixing a bushing (102) on the bunch (101) at a predetermined distance from an end (103) of the bunch (101);
overlapping and/or interlacing the wire filaments so that to form a net (104);
fixing another bushing (105) on the bunch (101), thereby to avoid unweaving of the net.

11. The method of claim 10 further including the step of forming a guide rod that includes cutting of a certain number of the wire filaments at the distal end; and twisting the remaining number of the filaments together.

12. A retrieval basket (20) for entrapping and retaining an object located in a body for its extraction therefrom, the basket comprising a structure having a proximal end (270) and a distal end (271) and constituted by a plurality of filaments extending from the proximal end towards the distal end, said filaments have portions that are bound together to define a plurality of strands (273 and 274) at the proximal end (270) each strand including a plurality of the filaments, said plurality of strands (273 and 274) ramify at corresponding branching points into loops having various shapes and sizes, at least a part of said loops are overlapped and/or interlaced so as to define a net at least at the distal end (271), thereby imparting structural rigidity and dilation abilities to the basket when opened;
**characterized in that** one of the strands (274) of said plurality of strands is common for all the wire filaments and forms a loop, and **in that** said structure has a spoon-like shape.

13. The basket of claim 12 wherein the net is formed of cells having a seize decreasing from the proximal end towards the distal end.

14. The basket of claim 12 wherein the structure is fabricated from a single length of wire.

15. The basket of claim 12 wherein the structure is fabricated from several wires.

16. The basket of claim 12 wherein at least a part of the filaments, which originate from the proximal end (21, 271), also arrive at the proximal end after - winding.

17. The basket of claim 12 wherein said filaments are bound together at said proximal end (270) to form a manipulation cable.

18. The basket of claim 12 wherein the filaments are.made of metallic material.

19. The basket of claim 18 wherein said metallic material has thermo-mechanical shape memory characteristic.

20. The basket of claim 18 wherein said metallic material has superelastic characteristic.

21. The basket of claim 18 wherein said metallic material is selected from NiTi basted alloy and stainless steel.

22. The basket of claim 12 wherein, the filaments are made of non-metallic material.

23. The basket of claim 22 wherein said non-metallic material is Capron.

24. The basket of claim 12 wherein the filaments are covered by a coating layer.

25. A surgical device for removing a foreign object from a body, comprising:
a retrieval basket (20) of any one of claims 12 to 24 for entrapping and retaining the object located in the body for its extraction therefrom; and
a basket control assembly coupled to the basket, comprising a tubular sheath adapted to penetrate into the body for reaching the object, a manipulator for manipulating the basket for extraction the object from the body, and a manipulation cable arranged within the sheath for connecting the basket to the manipulator, where the assembly is configured for retracting the basket within the sheath and protracting the basket therefrom for its opening.

26. The device of claim 25 wherein said manipulation cable is constituted by said plurality of filaments extending from said proximal end (270) towards the manipulator.

27. The device of claim 25 further comprising a filling tubing put at least on a portion of said manipulation cable.

28. The device of claim 25 wherein said manipulation cable is configured as a separate unit selected from a rod, cable and wire.

29. The device of claim 25 wherein a joint between a commun basket strand of the basket and the manipulation cable is established by means of a bushing.

30. The device of claim 25 wherein the filaments are made of metallic material having thermo-mechanical shape memory characteristic.

31. The device of claim 30 further comprising a controllable power supply source coupled to the filaments for passing electric current therethrough.

32. The device of claim 25 wherein said manipulation cable is constituted by said plurality of filaments extending from the basket towards the manipulator.

## Patentansprüche

1. Verfahren zur Herstellung eines Entnahmekorbs, der ein proximales Ende (270) und ein distales Ende (271) aufweist und aus einer Vielzahl von Drahtfasern ausgebildet ist, die sich von dem proximalen Ende zu dem distalen Ende erstrecken, wobei das Verfahren aufweist:
Auswählen von zumindest einem Draht;
Weben des Korbs aus dem Draht, wobei das Weben das Ausformen von Teilbereichen einschließt, die zusammengebunden werden, um eine Vielzahl von Litzen (273 und 274) an dem proximalen Ende (270) festzulegen, wobei jede Litze eine Vielzahl der Fasern umfasst, wobei die Vielzahl von Litzen (273 und 274) an entsprechenden Verzweigungspunkten in Schlaufen verzweigt, die verschiedene Formen und Größen aufweisen, wobei sich zumindest ein Teil der Schlaufen überlappt und/oder kreuzt, um so zumindest an dem distalen Ende (271) ein Netz zu bilden; und
Ausformen einer Form des Entnahmekorbs, dadurch Verleihung von struktureller Steifigkeit und Dilatationseignung für den Korb, wenn dieser geöffnet wird;
**dadurch gekennzeichnet, dass** das Weben so ausgeführt wird, dass eine der Litzen (274) der Vielzahl von Litzen (273 und 274) allen Drahtfasern gemeinsam ist und eine Schlaufe ausformt, und dadurch dass der Entnahmekorb in einer löffelartigen Form ausgeformt wird.

2. Verfahren nach Anspruch 1, das weiterhin das Zusammenbinden der Litzen an dem proximalen Ende aufweist, um ein Bedienungskabel (58) auszuformen.

3. Verfahren nach Anspruch 2, das weiterhin das Binden der Fasern des Bedienungskabels (58) durch temporäre Hülsen (59a, 59b) aufweist.

4. Verfahren nach Anspruch 3, das weiterhin das Härten des Entnahmekorbs aufweist.

5. Verfahren nach Anspruch 1, das das Zusammenbinden der Fasern an dem distalen Ende (271) aufweist, um eine Korbspitze auszuformen.

6. Verfahren nach Anspruch 1, wobei das Ausformen der Form das Anfertigen einer die Form ausformenden Aufspannvorrichtung (52) aufweist, die eine vorgegebene Form aufweist.

7. Verfahren nach Anspruch 1, wobei das Weben das Anfertigen einer Webeaufspannvorrichtung (52) aufweist, die ein vorgegebenes Muster aufweist, das aus Nuten ausgeformt wird, die auf einer Oberfläche der Eingerichtet sind.

8. Verfahren nach Anspruch 7, wobei zumindest ein Teil des Webens des Korbs auf der Webealufspannvorrichtung (52) ausgeführt wird, wobei das Weben aufweist:
Einspannen eines Endes des Drahtes, während das andere Ende auf den oberen Teil der Aufspannvorrichtung gelegt wird; und
Bewegen des freien Endes entlang der Nuten durch Wegnehmen von der Aufspannvorrichtung und Zurückbringen dorthin mit Überlappen und/oder Überkreuzen der Drahtfasern in Übereinstimmung mit einem erwünschten Muster des Webens und einer räumlichen Struktur der Korbstruktur.

9. Verfahren nach Anspruch 6, wobei das Ausformen der Form das Flechten der Fasern und der Litzen auf der die Form ausformenden Aufspannvorrichturg (52) aufweist.

10. Verfahren nach Anspruch 1, wobei das Weben des Korbes aufweist:
Auswählen einer vorgegebenen Anzahl von Drähten;
Zusammenfügen der Drähte zu einem Bündel (101);
Befestigen einer Hülse (102) auf dem Bündel (101) in einer vorgegebenen Entfernung von einem Ende (103) des Bündels (101);
Überlappen und/oder Kreuzen der Drahtfasern, so dass ein Netz (104) ausgeformt wird;
Befestigen einer weiteren Hülse (105) auf dem Bündel (101), wodurch das Entflechten des Netzes vermieden wird.

11. Verfahren nach Anspruch 10, das weiterhin den Schritt des Ausformen eines Führungsstabes aufweist und der das Abschneiden einer bestimmten Anzahl der Drahtfasern an dem distalen Ende aufweist; und das Zusammendrehen der verbleibenden Anzahl von Fasern.

12. Entnahmekorb (20) zur Aufnahme und Einbehaltung eines in einem Körper angeordneten Objekts zu dessen Extrahierung aus diesem, wobei der Korb eine Struktur ausweist, die ein proximales Ende (270) und ein distales Ende (211) aufweist und aus einer Vielzahl von Drahtfasern ausgebildet ist, die sich von dem proximalen Ende zu dem distalen Ende erstrecken, wobei die Fasern Teilbereiche aufweisen die zusammengebunden sind, um an dem proximalen Ende (270) eine Vielzahl von Litzen (27 und 274) festzulegen, wobei jede Litze eine Vielzahl der Fasern aufweist, wobei die Vielzahl von Litzen (273 und 274) an entsprechenden Verzweigungspunkten in Schlaufen verzweigt, die verschiedene Formen und Großen aufweisen, sich zumindest ein Teil der Schlaufen überlappt und/oder kreuzt, um so zumindest an dem distalen Ende (271) ein Netzt zu bilden, wodurch strukturelle Steifigkeit und Dilatationseignung für den Korb verliehen werden, dieser geöffnet wird;
**dadurch gekennzeichnet, dass** eine der Litzen (274) der Vielzahl von Litzen allen Drahtfasern gemeinsam ist und eine Schlaufe ausformt, und dadurch dass die Struktur eine löffelartige Form aufweist.

13. Korb nach Anspruch 12, wobei das Netz ausgeformt ist aus Zellen die eine Größe aufweisen, die sich von dem proximalen Ende in Richtung des distalen Ende vergrößert.

14. Korb nach Anspruch 12, wobei die Struktur aus einer einzelnen Länge an Draht hergestellt ist.

15. Korb nach Anspruch 12, wobei die Struktur aus mehreren Drähten hergestellt ist.

16. Korb nach Anspruch 12, wobei zumindest ein Teil der Fasern, die von dem proximalen Ende (21, 271) ausgehen, nach dem Wickeln auch an dem proximalen Ende eintreffen.

17. Korb nach Anspruch 12, wobei die Fasern an dem proximalen Ende (270) zusammengebunden sind, um ein Bedienungskabel auszuformen.

18. Korb nach Anspruch 12, wobei die Fasern aus einem metallischen Material hergestellt sind.

19. Korb nach Anspruch 18, wobei das metallische Material thermomechanische Formgedächtniseigenschaften aufweist.

20. Korb nach Anspruch 18, wobei das metallische Material superelastische Eigenschaften aufweist.

21. Korb nach Anspruch 18, wobei das metallische Material ausgewählt ist aus auf NiTi basierender Legierung und Edelstahl.

22. Korb nach Anspruch 12, wobei die Fasern aus nichtmetallischem Material hergestellt sind.

23. Korb nach Anspruch 22, wobei das nichtmetallische Material Capron ist.

24. Korb nach Anspruch 12, wobei die Fasern durch eine Beschichtungsschicht bedeckt sind.

25. Chirurgische Vorrichtung zur Entfernung eines Fremdkörpers aus einem Körper, die aufweist:
einen Entnahmekorb (20) nach einem der Ansprüche 12 bis 24 zur Aufnahme und Einbehaltung des in dem Körper angeordneten Objekts zu dessen Extrahierung aus diesem; und
eine mit dem Korb gekoppelte Korbsteueranordnung, die eine rohrförmige Umhüllung aufweist und eingerichtet ist in den Körper einzudringen um das Objekt zu erreichen, einen Manipulator zur Betätigung des Korbes zur Entfernung des Objekts aus dem Körper, und ein der Umhüllung angeordnetes Betätigungskabel um den Korb mit dem Manipulator zu verbinden, wobei die Anordnung eingerichtet ist zum Zurückziehen des Korbes innerhalb der Umhüllung und Herausziehen des Korbes aus dieser, um ihn zu öffnen.

26. Anordnung nach Anspruch 25, wobei das Betätigungskabel aus der Vielzahl von Fasern gebildet ist, die sich von dem proximalen Ende (270) zu dem Manipulator erstrecken.

27. Anordnung nach Anspruch 25, die weiterhin ein Füllrohr aufweist, das zumindest auf einen Teil des Betätigungskabels aufgebracht ist.

28. Anordnung nach Anspruch 25, das Betätigungskabel als eine getrennte Einheit ausgebildet ist, die ausgewählt ist aus einem Stab, einem Kabel und einem Draht.

29. Anordnung nach Anspruch 25, wobei eine Verbindung zwischen einer gemeinsamen Korblitze des Korbes und dem Betätigungskabel mittels einer Hülse eingerichtet ist.

30. Anordnung nach Anspruch 25, wobei die Fasern aus einem metallischen Material hergestellt sind, das eine thermomechanische aufweist.

31. Anordnung nach Anspruch 30, die weiterhin eine steuerbare Energieversorgungsquelle aufweist, die mit den Fasern gekoppelt ist, um elektrischen Strom durch diese zu leiten.

32. Anordnung nach Anspruch 25, wobei das Betätigungskabel aus der Vielzahl von Fasern gebildet ist, die sich von dem Korb zu dem Manipulator erstrecken.

## Revendications

1. Procédé de fabrication d'un panier de récupération ayant une extrémité proximale (270) et une extrémité distale (271), le panier étant constitué d'un ensemble de filaments de fil entre l'extrémité proximale vers l'extrémité distale,
procédé selon lequel :
- on sélectionne au moins un fil,
- on tisse le panier avec le fil, le tissage consistant à former des parties liées pour former un ensemble de cordons (273 et 274) à l'extrémité proximale (270), chaque cordon étant formé d'un ensemble de filaments, cet ensemble de filaments (273 et 274) se ramifiant à des points de branchement correspondants pour former des boucles ayant différentes formes et dimensions, au moins une partie des boucles se chevauchant et/ou étant imbriquée de façon à former un filet au moins à l'extrémité distale (271), et
- on réalise une forme de panier de récupération en donnant au panier, une rigidité de structure et une possibilité de dilatation à l'ouverture,
**caractérisé en ce que**
le tissage est tel qu'une extrémité des cordons (274) de l'ensemble des cordons (273 et 274) est commune à tous les filaments de fil et constitue une boucle et que le panier de récupération est en forme de cuillère.

2. Procédé selon la revendication 1,
selon lequel
on noue les cordons pour les réunir à l'extrémité proximale et former un câble de manipulation (58).

3. Procédé selon la revendication 2,
selon lequel
en outre, on noue les filaments du câble de manipulation (58) par des douilles provisoires (59a, 59b).

4. Procédé selon la revendication 3,
selon lequel
on recuit le panier de récupération.

5. Procédé selon la revendication 1,
selon lequel
on réunit les filaments par nouage à l'extrémité distale (271) pour former une extrémité de panier.

6. Procédé selon la revendication 1,
selon lequel
la mise en forme consiste à préparer un gabarit (52) selon une forme prédéfinie.

7. Procédé selon la revendication 1,
selon lequel
le tissage consiste à préparer un gabarit de tissage (52) ayant une forme prédéterminée par des rainures configurées à la surface du gabarit.

8. Procédé selon la revendication 7,
selon lequel
au moins une partie du tissage du panier se fait sur le gabarit de tissage (52) et le tissage comprend les opérations suivantes :
- fixation d'une extrémité du fil pendant que l'on place l'autre extrémité sur le dessus du gabarit, et
- déplacement de l'extrémité libre le long des rainures en l'extrayant du gabarit et retournement avec chevauchement et/ou imbrication des filaments de fil selon un motif prédéfini de tissage et de topologie de structure du panier.

9. Procédé selon la revendication 6,
selon lequel
la réalisation de la forme consiste à nouer les filaments et les cordons sur le gabarit de mise en forme (52).

10. Procédé selon la revendication 1,
selon lequel
le tissage du panier comprend les opérations suivantes :
- sélection d'un nombre prédéterminé de fils,
- réunir des fils en un faisceau (101),
- fixation d'une douille (102) sur le faisceau (101), à une distance prédéterminée d'une extrémité (103) du faisceau (101),
- chevauchement et/au imbrication des filaments de fil pour former un filet (104), et
- fixation d'une autre douille (105) sur le faisceau (101) pour éviter que le filet ne se défasse.

11. Procédé selon la revendication 10,
comprenant en outre l'étape de formation d'une tige de guidage consistant à couper un certain nombre de filaments de fil à l'extrémité distale et à tordre le nombre restant de filaments pour les réunir.

12. Panier de récupération (20) pour piéger et retenir un objet situé dans le corps humain afin de l'en extraire, le panier ayant une structure avec une extrémité proximale (270) et une extrémité distale (271), le panier étant constitué par un ensemble de filaments entre l'extrémité proximale et l'extrémité distale, les filaments ayant des parties nouées pour définir un ensemble de cordons (273 et 274), à l'extrémité proximale (270), chaque cordon ayant un ensemble de filaments, l'ensemble de cordons (273 et 274) se ramifiant en des points de branchement correspondants pour former des boucles ayant différentes formes et dimensions, au moins une partie des boucles se chevauchant et/ou étant imbriquées de façon à former un filet au moins à l'extrémité distale (271), pour donner la rigidité de structure et des caractéristiques de dilatation au panier ouvert,
**caractérisé en ce que**
l'un des cordons (274) parmi l'ensemble des cordons est commun à tous les filaments de fil et forme une boucle et la structure est en forme de cuillère.

13. Panier selon la revendication 12, dont le filet est formé de cellules ayant une dimension diminuant à partir de l'extrémité proximale vers l'extrémité distale.

14. Panier selon la revendication 12,
dans lequel
la structure est fabriquée à partir d'une seule longueur de fil.

15. Panier selon la revendication 12,
dans lequel
la structure est fabriquée à partir de plusieurs fils.

16. Panier selon la revendication 12,
dans lequel
au moins une partie des filaments issus de l'extrémité proximale (21, 271) revient à l'extrémité proximale après enroulement,

17. Panier selon la revendication 12,
dans lequel
les filaments sont noués les uns aux autres à l'extrémité proximale (270) pour former un câble de manipulation.

18. Panier selon la revendication 12,
dans lequel
les filaments sont en métal.

19. Panier selon la revendication 18,
dans lequel
le métal a une caractéristique de mémoire de forme thermomécanique.

20. Panier selon la revendication 18,
dans lequel
le métal a une caractéristique de superélasticité,

21. Panier selon la revendication 18,
dans lequel
le métal est choisi parmi des alliages à base de NiTi et d'acier inoxydable.

22. Panier selon la revendication 12,
dans lequel
les filaments sont en matière non métallique.

23. Panier selon la revendication 22,
dans lequel
la matière non métallique est le Capron.

24. Panier selon la revendication 12,
dans lequel
les filaments sont couverts d'une couche de revêtement.

25. Dispositif chirurgical pour extraire un objet étranger du corps humain, comprenant:
- un panier de récupération (20) selon les revendications 12 à 24 pour piéger et retenir l'objet localisé dans le corps afin de l'en extraire, et
- un dispositif de commande de panier, couplé au panier et comportant une gaine tubulaire susceptible d'être introduite dans le corps humain pour atteindre l'objet, un manipulateur pour manipuler le panier d'extraction de l'objet hors du corps humain et un câble de manipulation engagé dans là gaine pour relier le panier au manipulateur,
- l'assemblage étant configuré pour rétracter le panier dans la gaine et extraire le panier pour l'ouvrir.

26. Dispositif selon la revendication 25,
dans lequel
le câble manipulation est formé d'un ensemble de filaments s'étendant entre l'extrémité proximale (270) et le manipulateur.

27. Dispositif selon la revendication 25,
comprenant en outre un tubage de remplissage placé au moins sur une partie du câble de manipulation.

28. Dispositif selon la revendication 25,
dans lequel
le câble de manipulation est configuré comme unité distincte choisie parmi une tige, un câble ou un fil.

29. Dispositif selon la revendication 25,
dans lequel
un joint est prévu entre le cordon commun du panier et le câble de manipulation réalisé par l'intermédiaire d'une douille.

30. Dispositif selon la revendication 25,
dans lequel
les filaments sont en un métal ayant une caractéristique de mémoire de forme thermomécanique.

31. Dispositif selon la revendication 30, comprenant en outre une source d'alimentation électrique commandée, couplée aux filaments pour faire passer du courant.

32. Dispositif selon la revendication 25,
dans lequel
le câble de manipulation est formé d'un ensemble de filaments s'étendant entre le panier et le manipulateur.
